Europäisches Patentamt

European Patent Office

Office européen des brevets

(11) Publication number: **0 468 520 A2**

# EUROPEAN PATENT APPLICATION

(21) Application number: **91112601.9**

(51) Int. Cl.5: **A61K 31/70**

(22) Date of filing: **26.07.91**

(30) Priority: **27.07.90 JP 197778/90**

(43) Date of publication of application:
**29.01.92 Bulletin 92/05**

(84) Designated Contracting States:
**DE FR GB IT**

(71) Applicant: **MITSUI TOATSU CHEMICALS, Inc.**
**2-5 Kasumigaseki 3-chome**
**Chiyoda-Ku Tokyo 100(JP)**

(72) Inventor: **Tokunaga, Tohru**
**1-9-7, Horinouchi**
**Suginami-ku, Tokyo(JP)**
Inventor: **Kataoka, Tetsuro**
**2-47-10, Hijirigaoka**
**Tama-shi, Tokyo(JP)**
Inventor: **Yamamoto, Saburo**
**4-1-18, Naka-machi**
**Koganei-shi, Tokyo(JP)**
Inventor: **Kuramoto, Etsuro**
**Miyanodai-Aparto 640-15, 2141, Tougou**
**Mobara-shi, Chiba-ken(JP)**
Inventor: **Yano, Osamu**
**Miyanodai-daini-aparto 740-11, 2142, Tougou**
**Mobara-shi, Chiba-ken(JP)**
Inventor: **Shimada, Shizuo**
**Miyanodai-aparto 640-35, 2141, Tougou**
**Mobara-shi, Chiba-ken(JP)**
Inventor: **Makino, Tadashi**
**5-14-8, Hiyoshidai**
**Togane-shi, Chiba-ken(JP)**

(74) Representative: **Vossius & Partner**
**Siebertstrasse 4 P.O. Box 86 07 67**
**W-8000 München 86(DE)**

(54) **Immunostimulatory remedies containing palindromic DNA sequences.**

(57) Single-stranded, linear polydeoxyribonucleotides with a base number of 10 to 100 containing at least one structure represented by the following general formula:

$$5'-X_n \bullet\bullet\bullet X_3 X_2 X_1 Y_1 Y_2 Y_3 \bullet\bullet\bullet Y_n-3' \qquad (I)$$

( wherein n is an integer from 3 to 50; $X_1$, $X_2$, $X_3$, $\bullet\bullet\bullet$, $X_n$ and $Y_1$, $Y_2$, $Y_3$, $\bullet\bullet\bullet$, $Y_n$ are each a monodeoxyribonucleotide; $X_1$, $X_2$, $X_3$, $\bullet\bullet\bullet$ and $X_n$ may be the same or different nucleotides; and bases in $X_1$ and $Y_1$, in $X_2$ and $Y_2$, in $X_3$ and $Y_3$, in $\bullet\bullet\bullet$, and in $X_n$ and $Y_n$ are complementary with each other as defined by Watson & Crick ), and double-stranded, linear polydeoxyribonucleotides, in which at least one single-stranded, linear polydeoxyribonucleotide contains at least one structure represented by the general formula (I), both show strong immunostimulatory activity.

Thus, remedies containing, as active ingredient, such a specific polydeoxyribonuleotide as dsecribed above, or a salt thereof, are efficacious against malignant tumors, infectious diseases, immunodeficiency diseases and autoimmune diseases, with minimized side-effects.

This invention relates to immunostimulatory remedies capable of arresting and curing diseases susceptible to medicines having immunopharmacological activity, such as malignant tumors, infectious diseases, immunodeficiency diseases and autoimmune diseases, by modifying the function of the immune system.

Bacterial preparations, polysaccharides, a variety of low-molecular compounds such as levamisole, and various cytokines such as interferon, have been used or investigated as the immunostimulatory remedies against malignant tumors, infectious diseases, immunodeficiency diseases and autoimmune diseases. However, these remedies do not exhibit satisfactory therapeutic effects and cause a variety of unfavorable side effects. Hence, there has been a demand for highly efficacious remedies with minimized side effects.

On the other hand, some attempts have also been made to apply nucleic acids to the above purposes. The complex of polyinosinic acid and polycytidylic acid, which is a synthetic polyribonuleotide ( hereinafter abbreviated as RNA ), enhances the activity of mouse natural killer cells ( hereinafter abbreviated as NK cells ) which are members of the immune system, and promotes the production of interferon ( hereinafter abbreviated as IFN ) which is a regulatory substance in the immune system. It was also proved to be efficacious against experimental tumors presumably by immunological mechanism(s).

Djeu, J.Y., et al.; J. Immunol., 122, 175-181 ( 1979 )

Levy, H.B., et al.; Proc. Natl. Acad. Sci. U.S.A., 62, 357-361 ( 1969 )

It was reported that a complex of polyadenylic acid and polyuridylic acid ( a synthetic RNA ) is also efficacious against experimental tumors.

Lacour, F., et al.; Cancer Res., 32, 648-649 ( 1972 )

However, these RNAs also cause marked side effects, such as fever, and hence have not yet been put into practical use.

The present inventors formerly discovered that synthetic polydeoxyribonucleotides ( hereinafter abbreviated as DNAs ) have immunopharmacological activity and are efficacious against malignant tumors as synthetic RNAs are. ( Japanese Patent Kokai No.33222/1984 ). Unlike the synthetic RNAs, these synthetic DNAs may presumably be useful remedies because of their minimized side effects, such as fever, but involve the following problems.

(1) The molecular weight must be high ( 30000 Da or more ) to ensure a satisfactory pharmacological activity, and this requires enzymatic synthesis. The products thus obtained, when used as a drug, can contain enzymes left unremoved and are very unsatisfactory in terms of safety.

(2) It is difficult by enzymatic synthesis to accurately control the molecular-weight distribution of the products, and hence the molecular-weight distribution is generally different among production lots. This is unfavorable in terms of specification setting for drugs.

Accordingly, when a DNA is employed as a drug, it is most desirable to adopt the one prepared by chemical synthesis without using any enzyme, but it is difficult to synthesize a DNA having a molecular weight of 30000 Da or more at the present technological level.

On the other hand, low-molecular DNAs which can be easily synthesized chemically are generally low in pharmacological activity and are not generally useful as drugs.

The object of this invention is to provide immunostimulatory remedies containing, as the active ingredient, a chemically prepared DNA, which are high in safety and exhibit enhanced efficacy and usefulness as drugs.

To solve the problems described above, the present inventors had continued intensive studies by synthesizing DNAs with various base sequences, and found that DNAs carrying a specific kind of base sequences have strong immunostimulatory activity. This invention was accomplished on the basis of these findings.

Thus, this invention relates to immunostimulatory remedies containing, as the active ingredient, a single-stranded, linear polydeoxyribonucleotide with a base number of 10 to 100 containing at least one structure represented by the following general formula:

$$5'\text{-}X_n \bullet\bullet\bullet X_3 X_2 X_1 Y_1 Y_2 Y_3 \bullet\bullet\bullet Y_n\text{-}3' \qquad (I)$$

( wherein n is an integer from 3 to 50; $X_1$, $X_2$, $X_3$,••• , $X_n$ and $Y_1$, $Y_2$, $Y_3$,••• , $Y_n$ are each a monodeoxyribonucleotide; $X_1$, $X_2$, $X_3$, •••and $X_n$ may be the same or different nucleotides; and bases in $X_1$ and $Y_1$, in $X_2$ and $Y_2$, in $X_3$ and $Y_3$, in •••, and in $X_n$ and $Y_n$ are complementary with each other as defined by Watson & Crick ) or a salt thereof, and to immunostimulatory remedies containing, as the active ingredient, a double-stranded, linear polydeoxyribonucleotide or a salt thereof, in which at least one single-stranded, linear polydeoxyribonucleotide contains at least one structure represented by the general formula (I).

The structure represented by the general formula (I) of this invention is a sequence of monodeoxyribonucleotides called palindromic structure, in which $X_1$ and $Y_1$, $X_2$ and $Y_2$, $X_3$ and $Y_3$, $\bullet\bullet\bullet$, and $X_n$ and $Y_n$ are complementary with each other as defined by Watson & Crick. A palindromic structure generally means one of the symmetric structures found in a double-stranded DNA, and these structures are the recognition site for many kinds of restriction enzymes. In this invention, this structural nomenclature commonly employed for double-stranded DNAs is used also for single-stranded DNAs, for convenience, and the structure represented by the general formula (I) is hereinafter called the palindromic structure or palindromes.

What is to be noticed here is that the single- or double-stranded DNAs which are entirely composed of alternately repeated sequence of only two types of monodeoxynucleotides ( for example, G and C, or A and T ) cannot achieve the purpose of this invention. "n" in the general formula (I) is an integer from 3 to 50.

The following sequences are examples of the desirable structure of the present invention,wherein G is a deoxyguanylic acid, A is a deoxyadenylic acid, C is a deoxycytidylic acid and T is a deoxythymidylic acid, and wherein the left side is 5'-terminal and the right side is 3'-terminal in each sequence:

GAGCTC, TAGCTA, AGGCCT, CATATG, TGTACA, GTTAAC, GATATC,

GGGCCC, TTGCAA ( n = 3 )

GTAGCTAC, AAGGCCTT, GGATATCC, CAGGCCTG, GCATATGC, GTGTACAC,

AGTTAACT ( n = 4 )

AGTAGCTACT, GAAGGCCTTC, AGGATATCCT, GCAGGCCTGC,

AGCATATGCT ( n = 5 ) $\bullet\bullet\bullet\bullet\bullet\bullet$

To achieve the purpose of this invention, it is more desirable that one or more of the 5'-CG-3' structure be included in the structure represented by the general formula (I). As examples of such a structure, may be mentioned the following sequences:

CGATCG, ATCGAT, TCGCGA, AACGTT, GCGCGC, CGTACG, AGCGCT,

CGGCCG, GACGTC, GTCGAC, CGCGCG, ACGCGT, CACGTG ( n = 3 )

ACGATCGT, GATCGATC, ATCGCGAT, CAACGTTG, AGCGCGCT, ACGTACGT,

TAGCGCTA, ACGGCCGT, CGACGTCG, CGTCGACG ( n = 4 )

GACGATCGTC, CGATCGATCG, GATCGCGATC, GCAACGTTGC,

CAGCGCGCTG, GACGTACGTC, CTAGCGCTAG, GACGGCCGTC, ACGACGTCGT,

ACGTCGACGT, ACAACGTTGT ( n = 5 ) $\bullet\bullet\bullet\bullet\bullet\bullet\bullet$

The single-stranded, linear DNA of this invention is an unbranched DNA molecule in which each of the component monodeoxyribonucleotides is linked to the adjacent monodeoxyribonycleotide through a[5'-3'] phosphodiester bond. The single-stranded, linear DNAs carrying less than six bases, which fail to satisfy the general formula (I), are not satisfactory. As may be apparent from the Examples described later, the longer the chain length of single-stranded, linear DNA, the better will be the result. However, the purpose of this invention may be sufficiently achieved with a DNA with a base number in the range from l0 to l00, because

synthesis becomes more difficult with increasing chain length and the activity is enhanced very slightly with base number more than 45.

These single-stranded, linear DNAs contain at least one sequence represented by the general formula (I), and those containing two or more of such sequences may also be used, as required. In the latter case, the sequences contained may be the same with or different from each other, and there may be an intervening sequence ( or sequences ) between thes sequences. The sequences other than those of formula (I) in the single-stranded, linear DNAs may be of any type, but should most preferably be a repeated structure of deoxyguanylic acid as shown in the Examples described later.

If the entire base sequence of the single-stranded, linear DNAs is composed of alternately repeated sequence of only two kinds of monodeoxyribonucleotides, such as GC and AT, the purpose of this invention cannot be achieved as shown in the Examples. Hence, the base number of the DNAs should be in the range from 6 to l00, preferably from l0 to l00, most preferably from l0 to 80. DNAs with base numbers exceeding l00 should be avoided because chemical synthesis of such DNAs is difficult.

Double-stranded, linear DNA is a double helical complex composed of a single-stranded, linear DNA as described above [DNA(A)] and a second single-stranded, linear DNA [ DNA(B)] with base sequence which are partially or completely complementary with those of DNA(A). Either DNA(A), DNA(B) or the both must contain at least one sequence represented by the general formula (I). Such double-stranded, linear DNAs alone have the same immunostimulatory activity as single-stranded, linear DNAs do as shown in the Examples described later.

Needless to say, mixtures of a single-stranded linear DNA and a double-stranded, linear DNA are also included in this invention.

Single-stranded, linear DNAs can be synthesized by known methods using for instance a DNA synthesizer (for example, Model 380B of Applied Biosystems ) and, as substrate, deoxynucleoside-$\beta$-cyanoethylphosphamidite ( for example, a product of MilliGen/Biosearch, Division of Millipore ). The DNAs thus synthesized may be used as such, or may be purified, as required, by removing the by-products and unreacted substances to give products of higher purity. For a simple example of such purification process, the salt of DNA formed is precipitated from its solution by addition of two times the volume of cold ethanol; the dried precipitate is dissolved in water or in a neutral buffer solution containing a physiological concentration of an inorganic salt; and the solution thus obtained is used as a remedy. It is more preferable to purify the synthesized DNA by electrophoresis on a polyacrylamide gel or by column chromatography, because DNA with the desired base sequence is thus obtained at a higher purity. Use of other methods for chemical synthesis of DNA, such as phosphotriester method, may also give the same desirable result as herein described. Thus, the pharmacological activity of the single-stranded, linear DNAs of this invention depends only upon the base sequence and not upon the method of synthesis.

Double-stranded, linear DNAs can be obtained, for example, by the known method in which equimolar amounts of single-stranded, linear DNA(A) and DNA(B) as described above are mixed, heated and cooled. Another known method may also be used, in which a double-stranded, linear DNA is obtained by the use of a DNA polymerase using DNA(A) as the template.

These DNAs may also be used in the form of medicinally approved salts. For example, sodium salts can be obtained by adding sodium hydroxide to an aqueous solution of DNA of this invention to adjust the pH to 7, followed by lyophilization. These DNAs may also be used as a complex with a polycationic compound, such as poly-L-lysine ( hereinafter abbreviated as PLL ). Such complex can be prepared, for example, by mixing an aqueous solution of DNA of this invention with an aqueous solution of PLL so that the DNA-PLL weight ratio will be about 4:3.

The immunostimulatory remedies of this invention may be used alone or in combination with other therapeutic means against such diseases the outbreak of which can be suppressed, or the progress of which can be arrested or delayed, by the functions of the immune system. As examples of such diseases, may be mentioned, among others, malignant tumors, autoimmune diseases, immunodeficiency diseases and infectious diseases. Malignant tumors are diseases such as gastric cancer, colorectal cancer, breast cancer, skin cancer, liver cancer, uterine cancer, reticulosarcomas, lymphosarcomas, leukemias, lymphomas and like diseases. Autoimmune diseases are the diseases which are considered to result from impaired self-recognizing function of the immune system, such as rheumatoid arthritis, SLE, juvenile onset diabetes, multiple sclerosis, autoimmune hemolytic anemia and myasthenia gravis, which are considered to be effectively cured by drugs having immunopharmacological activity. Infectious diseases are the diseases caused by infection with bateria, viruses or protozoans, and are considered to be effectively cured by drugs having immunopharmacological activity ( such as interferon ). As shown in the Examples described later, DNAs of this invention are capable of inducing interferon and are therefore expected to effectively cure infectious diseases, especially viral diseases. Immunodeficiency diseases are the diseases in which the functions of immune system are suppressed or lost, such as agammaglobulinemia and acquired immunodeficiency syndromes. Among the patients of these diseases, the morbidity of infectious diseases and malignant tumors is high, thus adversely affecting recuperation. DNAs of this invention, which are efficacious against malignant tumors and are also capable of inducing interferon, are expected to encourage the recuperation of the patients suffering immunodeficiency diseases by curing the malignant tumors and infectious diseases which are likely to concur in these patients.

Single- and double-stranded, linear DNAs of this invention may be administerd to animal and human bodies subcutaneously, intravenously, intramuscularly, intratumorally, orally or into the rectum, and the suitable administration route should be selected case by case depending on the type of disease and the conditions of the patient. For example, intratumoral or subcutaneous administration is preferable in the case of malignant tumors. The proper dose to humans is l to l000 mg/day when administered into the rectum or orally, and 0.0l to l00 mg/day when administered subcutaneously, intravenously, intratumorally or intramuscularly. Administration should be repeated once or twice per one to seven days, preferably once per one or two days, and the frequency of administration may be varied and the period of administration may be further prolonged, as required.

When administering single- or double-stranded, linear DNAs of this invention to animal and human bodies subcutaneously, intravenously, intramuscularly or intratumorally, it is preferable to appply it in the form of an injection prepared by dissolving the DNA in an aqueous solution which is nearly neutral ( pH 5 to 8 ) with a physiological osmotic pressure. As examples of such an aqueous solution, may be mentioned the isotonic sodium chloride solution specified in Pharmacopoeia of Japan, and aqueous solutions containing salts, compounds, additives or diluents medicinally approved. The single- and double-stranded, linear DNAs of this invention may be used as an injection either in the form of an aqueous solution as described above or in the form of solid obtained by lyophylizing the same.

The single- and double-stranded, linear DNAs of this invention, when orally administered to animal and human bodies, may be used in the form of capsules, granules, pills, fine granules, tablets or syrup, as in the case of common drugs.

The fact that a specific base sequence in a DNA molecule has an important effect upon its immunostimulatory activity has not been known at all, and this is a completely new finding.

DNAs of this invention enhance the production of interferon and macrophage activating factor, thus activating NK cells and macrophages, also enhance the production of colony-stimulating factor, promote the proliferation of lymphocytes, and are therefore considered to exhibit a wide range of immunostimulatory activity. In addition, these DNAs proved to be very efficacious remedies against experimental tumors, and experimental models for immunodeficiency diseases and for autoimmune diseases, through their immunostimulatory activity. Furthermore, the acute toxicity of these DNAs is much lower than that of synthetic RNA; thus these DNAs are expected to be highly efficacious and useful remedies against malignant tumors, various auto-immune diseases, immunodeficiency diseases and infectious diseases.

EXAMPLES

Detailed below are Test Methods and Examples, in which guanine, adenine, cytosine and thymine ( bases contained in nucleic acids ) are abbreviated as G, A, C and T, respectively, and the nucleotide sequence in each DNA molecule is represented so that the left side is 5'-terminal and the right side is 3'-terminal. The base sequence in each DNA used in the Examples is shown in the Sequence List appended at the end of this specification. The complex of polyinosinic acid and polycytidylic acid ( hereinafter abbreviated as polyI:C ), which is a synthetic RNA used as the control remedy, was purchased from Yamasa Shoyu.

Test Method 1 ( In-vitro tests on the augmentation of mouse NK-cell activity, on the production of interferon, and on the production of macrophage activating factor )

The tests were performed according to the known method described in the following literature:
Yamamoto, S., et al.; Jpn. J. Cancer Res., 79, 866-873 ( 1988 )
provided that the NK-cell activity was measured by a four-hour $^{51}$Cr release assay using YAC-l cells as targets, and the result was expressed by the average of triplicate measurements and the standard deviation. The effector to target ratio was l00:l unless otherwise stated.

Test Method 2 ( Titration of colony-stimulating factor )

5

The titer of colony-stimulating factor in the serum of female BALB/C mouse to which DNA had been administered was measured by the known method described in the following literature:

Bradley, T.R. & Metcalf, D.; J. Exp. Biol. Med. Sci., 44, 287-300 ( 1966 )

Test Method 3 ( Measurement of the activity to enhance the proliferation of lymphocytes )

BALB/C mice ( female, six weeks old, specific pathogen-free ) purchased were bred for at least one day, and a solution of DNA in PBS was administered subcutaneously. These test animals were killed by cervical dislocation after one, two, three or seven days. Spleens were immediately removed aseptically, transferred to HBSS containing 2% FCS, and then forced to pass through a 80- or 100-mesh stainless-steel screen. The single cell suspension thus obtained was centrifuged for five minutes at 1400 rpm, and the cells precipitated were collected by removing the supernatant.

The cells were suspended in RPMI1640 medium containing 10% FCS and 25 $\mu$M 2-mercaptoethanol ( hereinafter referred to as "ME medium" ), and was centrifuged for five minutes at 1400 rpm. The cells were collected and suspended in ME medium, and an aliquot of the suspension was taken for the measurement of the cell concentration by the trypan blue dye-exclusion method.

The cell suspension was diluted with ME medium, and concanavalin A ( hereinafter abbreviated as ConA ) was added so that the cell and ConA concentrations were $5 \times 10^5$/ml and 4 $\mu$g/ml, respectively. Two tenth milliliters of the cell suspension was transferred to a 96-well plastic microplate and was cultured at 37°C for 48 hours under 5% $CO_2$. Twenty microliters of [$^3$H]-thymidine ( 0.37 MBq/ml, NET-027A; product of New-England Nuclear Co., Ltd. ) was added, and the culture was further continued for 20 hours.

The cultured cells were collected on a glass filter by the use of a cell harvester (product of Skatron Co., Ltd.) and dried, and the radioactivity incorporated into the cells was measured with a liquid scintillation counter using a liquid scintillator (Econofluor, NEF-941; product of New-England Nuclear Co., Ltd.). Triplicate experiments were conducted both in the presence and in the absence of ConA, and the ratio of the two averages - Stimulation Index ( S.I. ) - was calculated:

$$S.I. = \frac{\text{Radioactivity incorporated in the presence of ConA (cpm)}}{\text{Radioactivity incorporated in the absence of ConA (cpm)}}$$

Test Method 4 ( Measurement of antitumor activity against mouse tumors )

CDF1 mice ( female, six weeks old, specific pathogen-free ) purchased were bred for at least one day, and the hairs on the right abdominal region were removed over an area of 5mm square. Separately, ascitic IMC carcinoma cells were collected from the peritoneal cavity of CDF1 mouse and subjected to centrifugal washing with HBSS; the number of living cells contained was calculated by the trypan blue dye-exclusion method; HBSS was added to give a cell concentration of $5 \times 10^6$/ml; and 0.1 ml of the cell suspension was injected into the right flank of the mice.

Beginning from four days after the tumor implantation, a solution of DNA in PBS was repeatedly administered into and around the tumor every other day for a total of six times; the test animals were killed 30 days after the implantation; the tumors were cut out, and their weight was measured.

Test Method 5 ( Test on the efficacy against rat adjuvant arthritis )

Male SD rats ( seven weeks old ) were bred for one week. Separately, lyophilized dead cells of Mycobacterium tuberculosis ( Aoyama B strain; product of Difco Laboratories ) were suspended in sterilized liquid paraffin ( Merk & Co., Inc. ) at a concentration of 5 mg/ml, and 0.1 ml of this suspension was injected into a hind-leg pad of the above test rats under ether anesthesia to induce adjuvant arthritis. A solution of DNA in PBS was then administered subcutaneously 1, 3, 5, 7, 9 and 11 days after adjuvant administration.

The extent of secondary inflammation was determined by measuring the volume of footpad; the position 7 mm above the knee joint of the untreated hind leg was marked, and the footpad volume under this marked position was measured with a plesthymometer. Inflammation Index was defined as the fractional increase (%) of the footpad volume as compared to that on the day of adjuvant inoculation.

Test Method 6 ( Test of efficacy on MRL/MPJ-lpr mouse autoimmune disease model )

A solution of DNA in PBS was administered to MRL/MPJ-lpr mice ( female, six weeks old ) subcutaneously three times per week, and the amount of protein in the urine was calculated from the urine volume excreted during l6 hours and the protein concentration therein.

Test Method 7 ( Acute toxicity test in mice )

DNA or RNA was administered to ICR mice ( l0 mice per group ) intravenously (iv ) or intraperitoneally ( ip ), and the number of living mice was counted 24 hours after administration. Graphs were made in which the ratio of living mice was plotted against the amount of DNA or RNA administered, and the amount of DNA or RNA per body weight that will kill half of the mice was estimated - $LD_{50}$ ) mg/kg ).

Test Method 8 ( Test of efficacy against mice infected with LP-BM5 viruses )

Stock solution of LP-BM5 viruses ( 0.5 ml ) was injected intraperitoneally to each of C57BL/l0 mice ( five weeks old ). From the next day, mice were fed with drinking water ad libitum to which azidothymidine ( AZT) used as a control drug, was added at a concentration of 0.5 mg/ml. DNA was administered intraperitoneally every day in an amount of l mg as a solution in PBS. Five weeks after the virus infection, the spleen cells obtained from the mice were suspended in RPMIl640 medium containing l0% FCS at a cell concentration of l x $l0^7$/ml. After culturing the cells at 37$^\circ$C for 20 hours under 5% $CO_2$ in the presence of IL-2 ( l000 U/ml, a product of Genzyme Corp.), NK cell activity was measured.

Test Method 9 ( Quantitation of DNA )

DNA was dissolved in 0.2mM phosphate buffer ( pH 7.0 ), and the absorbance at 260 nm was measured with the same buffer used as reference. DNA concentration was determined by assuming that the concentration of DNA that gave an absorbance of 1 was 20 $\mu$g/ml.

Example 1 ( Preparation of tablets containing, as active ingredient, a single-stranded, linear DNA of this invention )

A mixture of 5 g of sodium salt of a single-stranded, linear DNA ( Sequence 1 ), 53 g of lactose, 50 g of corn starch and 35 g of crystalline cellulose was kneaded with a solution of 5 g hydroxypropylcellulose in l0 ml water to form granules, which were dried at 50ºC for four hours. Magnesium stearate ( 2 g ) was then admixed, and the mixture was compressed into tablets ( each weighing 200 mg ) by the use of a tabletting machine.

Example 2 ( Preparation of capsules containing, as active ingredient, a single-stranded, linear DNA of this invention )

A mixture of 5 g of sodium salt of a single-stranded, linear DNA ( Sequence 1 ), 124 g of lactose, 90 g of corn starch, 70 g of crystalline cellulose and 11 g of magnesium stearate was filled into hard gelatin capsules (300 mg in each ) by the use of a capsule filling machine, thus giving capsules.

Example 3 ( Preparation of parenteral injections containing, as active ingredient, a single-stranded, linear DNA of this invention )

One gram of sodium salt of a single-stranded, linear DNA ( Sequence 1 ) and 0.5 g of sodium chloride were dissolved in 1 liter of distilled water for injection, and the mixture was filtered and sterilized, thus giving injections.

Example 4

The fact that DNAs containing palindromic structure have stronger immunopharmacological activity than those containing no palindromic structure was demonstrated by the following experiments.

A single-stranded, linear DNA ( Sequence 1 ) with a base number of 45 containing a palindromic structure ( GACGTC ) had a strong activity to augment the mouse NK-cell activity ( Table 1 ). In contrast, a single-stranded, linear DNA ( Sequence 2 ) with a base number of 45 containing no palindromic structure had a weaker activity to augment the mouse NK-cell activity ( Table 1 ).

Table 1

| Test Sample | Number of Bases | Concn. | NK-Cell Activity |
|---|---|---|---|
| Control | | 0 | 14.3±1.3 |
| Sequence 1 | 45 | 10 | 26.4±2.6 |
| | | 50 | 56.8±3.4 |
| | | 100 | 65.3±4.1 |
| | | 500 | 60.4±3.9 |
| Sequence 2 | 45 | 10 | 14.1±1.1 |
| | | 50 | 15.0±2.0 |
| | | 100 | 20.0±2.2 |
| | | 500 | 17.9±2.1 |

"Concn." means the final concentration of DNA ( $\mu$g/ml ) during the mouse spleen cell culture.

"Control" means the case in which the spleen cells were cultured in a medium containing no DNA.

Comparison made between two DNA molecules ( Sequence 3 and Sequence 4 ) with a base number of 30 ( partial structures of Sequence 1 ) indicated that the DNA containing a palindromic structure ( Sequence 3 ) had a stronger activity to augment the NK-cell activity than the DNA containing no palindromic structure ( Sequence 4 ) ( Table 2 ).

Table 2

| Test Sample | Number of Bases | NK-Cell Activity |
|---|---|---|
| Control | | 14.8±0.4 |
| Sequence 1 | 45 | 57.1±2.2 |
| Sequence 3 | 30 | 45.9±2.1 |
| Sequence 4 | 30 | 15.0±1.4 |

Each DNA was added to spleen cells to give a final concentration of 60 $\mu$g/ml.

The DNA ( Sequence 5 ), which was of much the same structure as the DNA ( Sequence 3 ) except that the array of two adjacent nucleotides (GT) in the palindromic structure was replaced with an array of reversed nucleotide sequence (TG), had a weaker activity to augment the NK-cell activity than the DNA ( Sequence 3 ) ( Table 3 ). However, the DNA (Sequence 6 ), which was of much the same structure as the DNA ( Sequence 3 ) except that the array of two adjacent nucleotides (GT) in the portion other than the

8

palindromic structure was replaced with a reversed sequence of nucleotides (TG), had a strong activity to augment the NK-cell activity like the DNA ( Sequence 3 ) ( Table 3 ).

The DNA ( Sequence 7 ), which is of much the same structure as the DNA ( Sequence 3 ) except that one nucleotide (C) in the palindromic structure is lacking, had a markedly weaker activity to augment the NK-cell activity than the DNA ( Sequence 3 ) ( Table 3 ). However, the DNA ( Sequence 8 ), which is of much the same structure as the DNA ( Sequence 3 ) except that one nucleotide (C) in the portion other than the palindromic structure is lacking had a strong activity to augment the NK-cell activity like the DNA ( Sequence 3 ) ( Table 3 ).

The DNA ( Sequence 9 ), which is of much the same structure as the DNA ( Sequence 3 ) except that the palindromic structure is translocated to the 5'-terminal, had as strong an activity to augment the NK-cell activity as that of the DNA ( Sequence 3 ) ( Table 3 ); however, the DNA ( Sequence l0 ), in which the palindromic structure is translocated to the central position, had a stronger activity to augment the NK-cell activity than that of the DNA ( Sequence 3 ) ( Table 4 ).

Table 3

| Test Sample | Number of Bases | NK-Cell Activity |
|---|---|---|
| Control | | 14.1±2.0 |
| Sequence 3 | 30 | 48.8±2.6 |
| Sequence 5 | 30 | 15.8±1.5 |
| Sequence 6 | 30 | 45.3±2.3 |
| Sequence 7 | 29 | 15.4±1.4 |
| Sequence 8 | 29 | 50.9±1.9 |

Each DNA was added to spleen cells to a final concentration of 50 $\mu$g/ml.

Table 4

| Test Sample | Number of Bases | NK-Cell Activity |
|---|---|---|
| Control | | 14.2±0.9 |
| Sequence 3 | 30 | 43.4±2.1 |
| Sequence 9 | 30 | 45.8±2.6 |
| Sequence 10 | 30 | 51.4±2.8 |

Each DNA was added to spleen cells to a final concentration of 50 $\mu$g/ml.

It was demonstrated from the above results that the presence of the palindromic structure (GACGTC) has an important effect upon the DNA's activity to augment the NK-cell activity, and that a higher activity can be obtained if the palindromic structure is located in the central position of the DNA chain.

Example 5

9

The following experiments demonstrated that the same is true of palindromic structures other than the one shown in Example 1 (GACGTC); single-stranded, linear DNAs containing any of these palindromic structures exhibited a stronger immunopharmacological activity than DNAs not containing these structures.

The DNAs ( Sequences 11 and 12 ), which were of much the same structure as the DNA ( Sequence 3 ) except that the palindrome (GACGTC) was replaced with other types of palindromes (AGCGCT and AACGTT), had a strong activity to augment the NK-cell activity like the DNA ( Sequence 3 ) ( Table 5 ).

Table 5

| Test Sample | Number of Bases | NK-Cell Activity |
|---|---|---|
| Control | | 14.1±2.0 |
| Sequence 3 | 30 | 48.8±2.6 |
| Sequence 11 | 30 | 52.3±3.3 |
| Sequence 12 | 30 | 43.7±2.3 |

Each DNA was added to spleen cells to a final concentration of 50 $\mu$g/ml.

A single-stranded, linear DNA containing no palindromic structure ( Sequence 13) had only a weak activity to augment the NK-cell activity, while DNAs ( Sequence 14 through 20 ), which were of much the same structure as the DNA ( Sequence 13 ) except that a part of the base sequence was replaced with a palindromic structure each had a strong activity ( Table 6 ).

Table 6

| Test Sample | Palindrome | NK-Cell Activity |
|---|---|---|
| Control | | 14.3±0.4 |
| Sequence 3 | GACGTC | 46.2±1.5 |
| Sequence 13 | None | 14.4±1.0 |
| Sequence 14 | GACGTC | 49.1±1.6 |
| Sequence 15 | CGATCG | 48.7±0.9 |
| Sequence 16 | ATCGAT | 43.5±1.2 |
| Sequence 17 | TCGCGA | 44.3±0.9 |
| Sequence 18 | GCGCGC | 48.4±0.5 |
| Sequence 19 | CGTACG | 42.9±0.7 |
| Sequence 20 | CGGCCG | 43.5±0.9 |

Each DNA was added to spleen cells to a final concentration of 50 $\mu$g/ml.

10

Example 6

The following experiment demonstrated that DNAs containing a palindromic structure with a base number of 8 or 10 also have a high immunopharmacological activity. The DNA ( Sequence 21 ) containing a palindromic structure (GACGTC), and the DNAs ( Sequences 22 and 23 ) containing an expanded palindromic structure (GGACGTCC, CGGACGTCCG), had a strong activity to augment the NK-cell activity ( Table 7 ). However, the DNA ( Sequence 24 ) containing a curtailed GACGTC structure (ACGT) had only a low activity.

### Table 7

| Test Sample | Palindrome | NK-Cell Activity |
|---|---|---|
| Control | | $14.9 \pm 1.0$ |
| Sequence 24 | ACGT | $15.0 \pm 0.8$ |
| Sequence 21 | GACGTC | $42.3 \pm 1.1$ |
| Sequence 22 | GGACGTCC | $45.5 \pm 2.2$ |
| Sequence 23 | CGGACGTCCG | $52.6 \pm 1.5$ |

Each DNA was added to spleen cells to a final concentration of 50 $\mu$g/ml.

Example 7

The fact that the immunopharmacological activity of DNAs containing a palindromic structure depends on the molecular length was discovered from the results of the following experiments.

Comparison of the activity to augment the NK-cell activity among DNAs ( Sequences 25 through 32 ) with a palindromic structure in the central position and having a variety of molecular lengths ( number of bases: 6 to 80 ) showed that the activity was observed only in the DNAs having 10 or more bases, increased with the number of bases, and changed little when the number of bases exceeded 45 ( Table 8 ).

11

Table 8

| Test Sample | Number of Bases | NK-Cell Activity |
|---|---|---|
| Control | | 14.0±1.0 |
| Sequence 25 | 6 | 14.5±0.8 |
| Sequence 26 | 10 | 25.1±1.2 |
| Sequence 27 | 15 | 28.8±1.3 |
| Sequence 28 | 20 | 37.9±1.2 |
| Sequence 29 | 30 | 45.6±2.1 |
| Sequence 30 | 45 | 55.3±1.8 |
| Sequence 31 | 60 | 55.0±2.0 |
| Sequence 32 | 80 | 57.1±2.4 |

Each DNA was added to spleen cells to a final concentration of 50 µg/ml.

Example 8

The immunopharmacological activity was observed also in DNAs with multiple palindromic structures.

This was demonstrated by testing the activity of the DNAs (Sequences 33 through 36 ) to augment the NK-cell activity. These DNAs were of much the same structure as the DNA ( Sequence 1 ) except that part of the structure was replaced with at least one palindromic structures (GACGTC) ( Table 9 ).

Table 9

| Test Sample | Number of Palindromes | NK-Cell Activity |
|---|---|---|
| Control | | 14.2±0.6 |
| Sequence 1 | 1 | 27.1±2.3 |
| Sequence 33 | 2 | 32.3±2.5 |
| Sequence 34 | 3 | 39.0±2.5 |
| Sequence 35 | 4 | 46.1±3.0 |
| Sequence 36 | 5 | 50.5±4.2 |

Each DNA was added to spleen cells to a final concentration of 20 µg/ml.

The DNAs ( Sequences 37 through 39 ) composed of repeated palindromic structures also had the activity to augment the NK-cell activity ( Table 10 ).

Table 10

| Test Sample | Palindrome | NK-Cell Activity |
|---|---|---|
| Control | | 14.2±0.5 |
| Sequence 37 | GACGTC | 45.1±3.2 |
| Sequence 38 | CACGTG | 47.6±2.9 |
| Sequence 39 | AACGTT | 42.5±3.2 |

Each DNA was added to spleen cells to a final concentration of 20 $\mu$g/ml.

Example 9

The palindrome-containing DNA which carries only guanine and cytosine as component base ( Sequence 40 ) and the one which carries only adenine and thymine as component base ( Sequence 41 ) had only a weak activity to augment the NK-cell activity ( Table 11 ).

Table 11

| Test Sample | Palindrome | Component Base | NK-Cell Activity |
|---|---|---|---|
| Control | | | 14.4±1.6 |
| Sequence 3 | GACGTC | G, A, C, T | 46.3±2.5 |
| Sequence 40 | GCGCGC | G, C | 14.9±1.3 |
| Sequence 41 | ATATAT | A, T | 14.5±1.4 |

Each DNA was added to spleen cells to a final concentration of 50 $\mu$g/ml.

It was demonstrated from the above results that, in order for a DNA to exhibit a satisfactory immunopharmacological activity, it must contain at least one palindrome composed of six or more bases; the total number of bases contained therein must be ten or more; and the sequences entirely composed of repetition of GC- or AT-array are unfavorable.

Example 10

Single-stranded, linear DNAs containing palindromic structure induced interferon ( hereinafter abbreviated as IFN ) and macrophage activating factor ( hereinafter abbreviated as MAF ).

The DNA ( Sequence 1 ) containing a palindromic structure with a base number of 45 had an in-vitro activity to induce IFN and MAF from mouse spleen cells, but the activity of the DNA ( Sequence 2 ) without palindromic structure was weaker ( Table 12 ).

Table 12

| Test Sample | Number of Bases | Concn. | IFN Titer (IU/ml) | MAF Activity (%) |
|---|---|---|---|---|
| Control | | 0 | <5 | 8.0±0.5 |
| Sequence 1 | 45 | 10 | 56 | 15.2±1.1 |
| | | 100 | 362 | 25.1±2.3 |
| Sequence 2 | 45 | 10 | 10 | 8.2±0.7 |
| | | 100 | 22 | 10.9±0.6 |

"Concn." indicates the final concentration ($\mu g/ml$) of DNA added to mouse spleen cells.

Example 11

Single-stranded, linear DNAs containing palindromic structure had an activity to induce colony stimulating factor ( hereinafter abbreviated as CSF ). An intravenous administration of the DNA ( Sequence 1 ) containing a palindromic structure with a base number of 45 caused the increase of the serum CSF titer, which reached its peak six hours after administration ( Table 13 ). No such activity was observed with the DNA ( Sequence 2 ) without palindromic structure.

Table 13

| Test Sample | Time after Administration ( hour ) | Number of Colonies ( x $10^{-5}$ cells ) |
|---|---|---|
| Control | 0 | 2 |
| Sequence 1 | 1 | 4 |
| | 3 | 10* |
| | 6 | 35** |
| | 12 | 12* |
| | 24 | 3 |
| Sequence 2 | 1 | 2 |
| | 3 | 2 |
| | 6 | 3 |
| | 12 | 5 |
| | 24 | 1 |

Each DNA was administered in an amount of 5mg.

*: $p < 0.05$, **: $p < 0.01$ ( by Student's t-test )

Example l2

ConA-stimulated proliferation of spleen cells was promoted in mice to which 5 mg of the palindrome-containing DNA ( Sequence 1 ) with a base number of 45 had been administered ( Table l4 ). However, no such activity was observed with the DNA ( Sequence 2 ) without palindromic structure.

Table 14

| Test Sample | Days after Administration | S.I. |
|---|---|---|
| Control | | 42.3 |
| Sequence 1 | 1 | 76.1** |
| | 2 | 66.7* |
| | 3 | 47.9* |
| Sequence 2 | 1 | 45.5 |
| | 2 | 43.1 |
| | 3 . | 42.0 |

*: $p < 0.05$, **: $p < 0.01$ ( by Student's t-test )

The above results demonstrated that DNAs containing palindromic structure exhibit a variety of immnopharmacological activities.

Example l3

When the palindrome-containing DNA ( Sequence 1 ) with a base number of 45 was administered to mice bearing IMC carcinoma tumors, dose-dependent suppression of the tumor weight ( namely, antitumor activity ) was observed ( Table l5 ). However, the DNA ( Sequence 2 ) with a base number of 45 without palindromic structure had only a weak antitumor activity ( Table 15 ).

Table 15

| Test Sample | Dose ($\mu$g) | Tumor Weight (g±SD) | % Suppression |
|---|---|---|---|
| Control | 0 | 2.55±1.20 | 0 |
| Sequence 1 | 10 x 6 | 1.85±0.91 | 27 |
| | 100 x 6 | 0.25±0.62 | 90 |
| Sequence 2 | 10 x 6 | 2.46±1.30 | 4 |
| | 100 x 6 | 2.15±1.16 | 16 |

IMC carcinoma cells were implanted to CDF1 mice (each group consisting of eight heads), and, beginning from four days after implantation, the test samples were administered intratumorally every other day for a total of six times. The tumor weitht was measured 30 days after implantation.

Example 14

The single-stranded, linear DNA ( Sequence 1 ) containing a palindromic structure was effective in suppressing the secondary inflammation in rat adjuvant arthritis model ( Table 16 ), while no such activity was observed with the DNA ( Sequence 2 ) without palindromic structure.

Table 16

| Test Sample | Inflammation Index (%) | | | | |
|---|---|---|---|---|---|
| | 10 Days | 14 Days | 17 Days | 21 Days | 28 Days |
| Control | 9.8±2.0 | 43.7±5.2 | 55.8±8.1 | 65.7±8.4 | 59.1±8.7 |
| Sequence 1 | 2.1±1.3[**] | 30.3±3.2[*] | 33.2±5.5[*] | 49.2±5.6[*] | 52.8±9.3 |
| Sequence 2 | 8.2±2.5 | 42.2±5.3 | 54.7±7.6 | 62.2±8.2 | 56.1±5.3 |

The extent of secondary inflammation after adjuvant injection is expressed by the fractional increase in footpad volume.

The dose of DNA was 2 mg/rat.  *: p<0.05, **: p<0.01 ( Student's t-test )

Example I5

Administration of the single-stranded, linear DNA ( Sequence 1 ) containing a palindromic structure to the autoimmune disease model, MRL/MPJ-lpr mice which suffer spontaneous outbreak of such diseases, suppressed the amount of protein excreted in the urine ( Table I7 ). However, no such activity was observed with the DNA ( Sequence 2 ) without palindromic structure.

Table 17

| Test Sample | Dose (mg/kg) | Protein Content in the Urine (mg) | |
|---|---|---|---|
| | | At the Start | After 4 Weeks |
| Control | 0 | 0.7±0.5 | 4.0±2.6 |
| Sequence 1 | 0.03 | 0.7±0.5 | 3.1±1.8 |
| | 0.3 | 0.7±0.5 | 3.3±1.8 |
| | 3 | 0.6±0.4 | 3.5±1.3 |
| Sequence 2 | 0.03 | 0.7±0.6 | 4.1±2.3 |
| | 0.3 | 0.5±0.4 | 3.5±1.6 |
| | 3 | 0.7±0.5 | 3.9±3.0 |

Table 17 ( contd. )

| Test Sample | Dose (mg/kg) | Protein Content in the Urine (mg) | |
|---|---|---|---|
| | | After 8 Weeks | After 12 Weeks |
| Control | 0 | 7.1±13.5 | 13.6±22.2 |
| Sequence 1 | 0.03 | 2.5±3.1 | 4.5±6.1 |
| | 0.3 | 1.7±1.1* | 8.6±12.3 |
| | 3 | 3.2±5.8 | 9.7±16.1 |
| Sequence 2 | 0.03 | 7.3±9.5 | 12.6±6.2 |
| | 0.3 | 5.8±5.2 | 10.7±12.3 |
| | 3 | 5.4±4.0 | 13.0±18.8 |

*: $p < 0.05$ ( Student's t-tgest )

The above results demonstrated that synthetic DNAs containing palindromic structure have not only immunopharmacological activities but also therapeutic effects on various diseases which are known to be susceptible to drugs with immunopharmacological activity.

Example 16

There was little difference in the activity to augment the NK-cell activity between the single-stranded, linear DNA ( Sequence 1 ) and the double-stranded, linear DNA ( Sequence 42 ) which was composed of the above DNA ( Sequence 1 ) and a second single-stranded, linear DNA with a complementary sequence to that of the former ( Table I8 ).

Table 18

| Test Sample | Concn. ($\mu$g/ml) | NK-Cell Activity |
|---|---|---|
| Control | 0 | 14.2±0.5 |
| Sequence 1 | 10 | 21.6±1.2 |
| | 100 | 44.0±2.1 |
| Sequence 42 | 10 | 19.9±1.5 |
| | 100 | 47.5±3.7 |

Example 17

Among the DNAs ( Sequences 43 through 48 ) containing the same palindromic structure (GACGTC), the DNA ( Sequence 43 ), whose sequence other than the palindromic structure was a simple repetition of deoxyguanylic acid, had the strongest activity to augment the NK-cell activity ( Table I9 ).

Table 19

| Test Sample | Repeated Unit in the Portion other than Palindromic Structure | NK-Cell Activity |
|---|---|---|
| Control | | 13.3±0.9 |
| Sequence 43 | G | 54.4±2.4 |
| Sequence 44 | A | 32.1±1.7 |
| Sequence 45 | T | 28.9±1.2 |
| Sequence 46 | C | 16.5±0.9 |
| Sequence 47 | GC | 25.0±1.0 |
| Sequence 48 | GA | 26.8±1.1 |

Each DNA was added to spleen cells to a final concentration of 50 $\mu$g/ml.

Example 18

DNAs containing the 5'-CG-3' structure in the palindromic structure had a stronger activity to augment the NK-cell activity than those without the 5'-CG-3' structure ( Table 20 ).

18

Table 20

| Test Sample | Palindrome | NK-Cell Activity |
|---|---|---|
| Control | | 12.8±0.6 |
| Sequence 11 | AACGTT | 50.0±1.8 |
| Sequence 12 | AGCGCT | 48.2±1.9 |
| Sequence 49 | CGATCG | 47.7±1.4 |
| Sequence 50 | ATCGAT | 47.0±1.6 |
| Sequence 51 | TCGCGA | 47.0±2.0 |
| Sequence 52 | GCGCGC | 46.9±1.2 |
| Sequence 53 | CGTACG | 46.6±1.9 |
| Sequence 54 | AGCGCT | 45.7±1.0 |
| Sequence 55 | CGGCCG | 44.2±1.7 |
| Sequence 3 | GACGTC | 42.1±1.5 |
| Sequence 56 | GTCGAC | 42.0±1.3 |
| Sequence 57 | CGCGCG | 40.1±1.4 |
| Sequence 58 | ACGCGT | 39.5±1.5 |
| Sequence 59 | AAGCTT | 20.1±0.8 |
| Sequence 60 | TTATAA | 19.9±0.5 |
| Sequence 61 | TATATA | 18.8±0.4 |
| Sequence 62 | AGTACT | 18.0±0.7 |
| Sequence 63 | GAATTC | 17.7±0.6 |
| Sequence 64 | CTGCAG | 17.5±0.5 |
| Sequence 65 | AAATTT | 17.5±0.7 |
| Sequence 66 | CCTAGG | 17.0±0.7 |

Each DNA was added to spleen cells to a final concentration of 50 $\mu$g/ml.

Example I9

In mice infected with LP-BM5 viruses ( model animals for immunodeficiency diseases ), the NK-cell activity of the lymphocytes was not enhanced by IL-2 stimulation unlike in normal mice; however, in the virus-infected mice to which the single-stranded, linear DNA ( Sequence 1 ) containing a palindromic structure had been administered, IL-2 was able to enhance the NK-cell activity of the lymphocytes ( Table 2l ). The degree of enhancement was nearly the same as that observed in the IL-2-stimulated lymphocytes of normal mice, and the IL-2-stimulated lymphocytes of infected mice to which AZT ( azidothymidine, a remedy for AIDS ) had been administered. But no such effect was observed with the single-stranded, linear DNA ( Sequence 2 ) without palindromic structure.

Table 21

| Mouse | Administered Remedy | IL-2 Addition | NK-Cell Activity |
|---|---|---|---|
| Normal | None | − | 7.0±0.9 |
| | | + | 23.5±1.5 |
| Infected | Control (PBS) | − | 1.1±0.4 |
| | | + | 1.1±0.2 |
| Infected | Sequence 1 | − | 5.9±0.8 |
| | | + | 24.1±1.4 |
| Infected | Sequence 2 | − | 3.3±0.9 |
| | | + | 3.5±0.7 |
| Infected | AZT | − | 1.9±1.0 |
| | | + | 21.4±0.6 |

Example 20

The single-stranded, linear DNA ( Sequence 1 ) containing a palindromic structure augmented the NK-cell activity and induced interferon even in SCID mice ( model animals for severe combined immunodeficiency ), but no such effect was observed with the single-stranded, linear DNA ( Sequence 2 ) without palindromic structure ( Table 22 ).

20

Table 22

| Mouse | Test Sample | NK-Cell Activity | IFN Titer (U/ml) |
|---|---|---|---|
| BALB/c | Control | 5.2±0.5 | <4 |
| | Sequence 1 | 15.4±0.7 | 128 |
| | Sequence 2 | 5.4±0.3 | <4 |
| SCID | Control | 18.3±1.0 | 16 |
| | Sequence 1 | 43.9±1.5 | 256 |
| | Sequence 2 | 20.0±1.1 | 16 |

Each DNA was added to spleen cells to a final concentration of 20 $\mu$g/ml.

NK-cell activity was measured at an E:T ratio of 25:1.

It was demonstrated from the results obtained in Examples 19 and 20 that the single-stranded, linear DNAs of this invention containing palindromic structure are capable of restoring ( at least partially ) the immunological functions of immunodeficiency disease model mice.

Example 21

Acute toxicity of the synthetic DNAs ( Sequences 1 and 3 ) with a base number of 45 and 30, respectively, was remarkably low as compared to that of the synthetic RNA (polyI:C) used as control ( Table 23 ).

Table 23

| Test Sample | Administration Route | $LD_{50}$ (mg/kg) |
|---|---|---|
| DNA ( Sequence 1 ) | iv | >500 |
| | ip | >1000 |
| DNA ( Sequence 3 ) | iv | >500 |
| | ip | >1000 |
| polyI:C | iv | 8 |
| | ip | 30 |

In addition, intraperitoneal administration of each of the synthetic DNAs ( Sequences 10, 11, 12, 14, 15, 16, 17, 18, 19, 20, 21, 22, 23, 24, 26, 27, 28, 29, 30, 31, 32, 33, 34, 35, 36, 37, 38, 39, 42, 43, 44, 45, 49, 50, 51, 52, 53, 54, 55, 56, 57 and 58 ) to DDY mice ( each group consisting of ten heads ), caused no death or body weight loss in any of the mice tested in one-week observation period.

EP 0 468 520 A2

SEQUENCE LISTING

Sequence No.: 1

Length of sequence: 45

Type of sequence: Nucleic acid

Type of Chain : Single-stranded

Topology: Linear

Kind of sequence: Other nucleic acid; synthetic DNA

Features of sequence: P: Contains palindrome (GACGTC)

ACCGATGACG TCGCCGGTGA CGGCACCACG ACGGCCACCG TGCTG


Sequence No.: 2

Length of sequence: 45

Type of sequence: Nucleic acid

Type of Chain : Single-stranded

Topology: Linear

Kind of sequence: Other nucleic acid; synthetic DNA

Features of sequence:

AAAAGAAGTG GGGTGCCCCC ACGATCACCA ACGATGGTGT GTCCA


Sequence No.: 3

Length of sequence: 30

Type of sequence: Nucleic acid

Type of Chain : Single-stranded

Topology: Linear

Kind of sequence: Other nucleic acid; synthetic DNA

Features of sequence: P: Contains palindrome (GACGTC)

ACCGATGACG TCGCCGGTGA CGGCACCACG

22

Sequence No.: 4

Length of sequence: 30

Type of sequence: Nucleic acid:

Type of Chain : Single-stranded

Topologty: Linear

Kind of sequence: Other nucleic acid; synthetic DNA

Features of sequence:

GGTGACGGCA CCACGACGGC CACCGTGCTG


Sequence No.: 5

Length of sequence: 30

Type of sequence: Nucleic acid

Type of Chain : Single-stranded

Topologty: Linear

Kind of sequence: Other nucleic acid; synthetic DNA

Features of sequence:

ACCGATGACT GCGCCGGTGA CGGCACCACG


Sequence No.: 6

Length of sequence: 30

Type of sequence: Nucleic acid

Type of Chain : Single-stranded

Topologty: Linear

Kind of sequence: Other nucleic acid; synthetic DNA

Features of sequence: P: Contains palindrome (GACGTC)

ACCGATGACG TCGCCGTGGA CGGCACCACG


23

Sequence No.: 7

Length of sequence: 29

Type of sequence: Nucleic acid

Type of Chain : Single-stranded

Topologty: Linear

Kind of sequence: Other nucleic acid; synthetic DNA

Features of sequence:

ACCGATGAGT CGCCGGTGAC GGCACCACG


Sequence No.: 8

Length of sequence: 29

Type of sequence: Nucleic acid

Type of Chain : Single-stranded

Topologty: Linear

Kind of sequence: Other nucleic acid; synthetic DNA

Features of sequence: P: Contains palindrome (GACGTC)

ACCGATGACG TCGCCGGTGA CGGCACCAG


Sequence No.: 9

Length of sequence: 30

Type of sequence: Nucleic acid

Type of Chain : Single-stranded

Topologty: Linear

Kind of sequence: Other nucleic acid; synthetic DNA

Features of sequence: P: Contains palindrome (GACGTC)

GACGTCGCCG GTGACGGCAC CACGACCGAT

Sequence No.: 10

Length of sequence: 30

Type of sequence: Nucleic acid

Type of Chain : Single- stranded

Topologty: Linear

Kind of sequence: Other nucleic acid; synthetic DNA

Features of sequence: P: Contains palindrome (GACGTC)

ACCACGACCG ATGACGTCGC CGGTGACGGC


Sequence No.: 11

Length of sequence: 30

Type of sequence: Nucleic acid

Type of Chain : Single- stranded

Topologty: Linear

Kind of sequence: Other nucleic acid; synthetic DNA

Features of sequence: P: Contains palindrome (AACGTT)

ACCGATAACG TTGCCGGTGA CGGCACCACG


Sequence No.: 12

Length of sequence: 30

Type of sequence: Nucleic acid

Type of Chain : Single- stranded

Topologty: Linear

Kind of sequence: Other nucleic acid; synthetic DNA

Features of sequence: P: Contains palindrome (AGCGCT)

ACCGATAGCG CTGCCGGTGA CGGCACCACG

Sequence No.: 13

Length of sequence: 30

Type of sequence: Nucleic acid

Type of Chain : Single-stranded

Topologty: Linear

Kind of sequence: Other nucleic acid; synthetic DNA

Features of sequence:

TCGGTGCAGG GAATGTCGCA GGACCCGGTC


Sequence No.: 14

Length of sequence: 30

Type of sequence: Nucleic acid

Type of Chain : Single-stranded

Topologty: Linear

Kind of sequence: Other nucleic acid; synthetic DNA

Features of sequence: P: Contains palindrome (GACGTC)

TCGGTGGACG TCATGTCGCA GGACCCGGTC


Sequence No.: 15

Length of sequence: 30

Type of sequence: Nucleic acid

Type of Chain : Single-stranded

Topologty: Linear

Kind of sequence: Other nucleic acid; synthetic DNA

Features of sequence: P: Contains palindrome (CGATCG)

TCGGTGCGAT CGATGTCGCA GGACCCGGTC

Sequence No.: 16

Length of sequence: 30

Type of sequence: Nucleic acid

Type of Chain : Single- stranded

Topologty: Linear

Kind of sequence: Other nucleic acid; synthetic DNA

Features of sequence: P: Contains palindrome (ATCGAT)

TCGGTGATCG ATATGTCGCA GGACCCGGTC


Sequence No.: 17

Length of sequence: 30

Type of sequence: Nucleic acid

Type of Chain : Single- stranded

Topologty: Linear

Kind of sequence: Other nucleic acid; synthetic DNA

Features of sequence: P: Contains palindrome (TCGCGA)

TCGGTGTCGC GAATGTCGCA GGACCCGGTC


Sequence No.: 18

Length of sequence: 30

Type of sequence: Nucleic acid

Type of Chain : Single- stranded

Topology: Linear

Kind of sequence: Other nucleic acid; synthetic DNA

Features of sequence: P: Contains palindrome (GCGCGC)

TCGGTGGCGC GCATGTCGCA GGACCCGGTC

Sequence No.: 19

Length of sequence: 30

Type of sequence: Nucleic acid

Type of Chain : Single-stranded

Topology: Linear

Kind of sequence: Other nucleic acid; synthetic DNA

Features of sequence: P: Contains palindrome (CGTACG)

TCGGTGCGTA CGATGTCGCA GGACCCGGTC

Sequence No.: 20

Length of sequence: 30

Type of sequence: Nucleic acid

Type of Chain : Single-stranded

Topology: Linear

Kind of sequence: Other nucleic acid; synthetic DNA

Features of sequence: P: Contains palindrome (CGGCCG)

TCGGTGCGGC CGATGTCGCA GGACCCGGTC

Sequence No.: 21

Length of sequence: 30

Type of sequence: Nucleic acid

Type of Chain : Single-stranded

Topology: Linear

Kind of sequence: Other nucleic acid; synthetic DNA

Features of sequence: P: Contains palindrome (GACGTC)

AAAAGAAGTG GGGACGTCTT ACGATCACCA

Sequence No.: 22

Length of sequence: 30

Type of sequence: Nucleic acid

Type of Chain : Single- stranded

Topology: Linear

Kind of sequence: Other nucleic acid; synthetic DNA

Features of sequence: P: Contains palindrome (GGACGTCC)

AAAAGAAGTG GGGACGTCCT ACGATCACCA

Sequence No.: 23

Length of sequence: 30

Type of sequence: Nucleic acid

Type of Chain : Single- stranded

Topology: Linear

Kind of sequence: Other nucleic acid; synthetic DNA

Features of sequence: P: Contains palindrome (CGGACGTCCG)

AAAAGAAGTG CGGACGTCCG ACGATCACCA

Sequence No.: 24

Length of sequence: 30

Type of sequence: Nucleic acid

Type of Chain : Single- stranded

Topology: Linear

Kind of sequence: Other nucleic acid; synthetic DNA

Features of sequence: P: Contains palindrome (ACGT)

AAAAGAAGTG GGAACGTCTT ACGATCACCA

Sequence No. 25

Length of sequence: 6

Type of sequence: Nucleic acid

Type of Chain : Single-stranded

Topology: Linear

Kind of sequence: Other nucleic acid; synthetic DNA

Features of sequence: P: Contains palindrome (GACGTC)

GACGTC


Sequence No.: 26

Length of sequence: 10

Type of sequence: Nucleic acid

Type of Chain : Single-stranded

Topology: Linear

Kind of sequence: Other nucleic acid; synthetic DNA

Features of sequence: P: Contains palindrome (GACGTC)

ATGACGTCGC


Sequence No.: 27

Length of sequence: 15

Type of sequence: Nucleic acid

Type of Chain : Single-stranded

Topology: Linear

Kind of sequence: Other nucleic acid; synthetic DNA

Features of sequence: P: Contains palindrome (GACGTC)

CCGATGACGT CGCCG

Sequence No.: 28

Length of sequence: 20

Type of sequence: Nucleic acid

Type of Chain : Single-stranded

Topology: Linear

Kind of sequence: Other nucleic acid; synthetic DNA

Features of sequence: P: Contains palindrome (GACGTC)

GACCGATGAC GTCGCCGGTG

Sequence No.: 29

Length of sequence: 30

Type of sequence: Nucleic acid

Type of Chain : Single-stranded

Topology: Linear

Kind of sequence: Other nucleic acid; synthetic DNA

Features of sequence: P: Contains palindrome (GACGTC)

TGACAGACCG ATGACGTCGC CGGTGGACGG

Sequence No.: 30

Length of sequence: 45

Type of sequence: Nucleic acid

Type of Chain : Single-stranded

Topology: Linear

Kind of sequence: Other nucleic acid; synthetic DNA

Features of sequence: P: Contains palindrome (GACGTC)

CCAGTGATTG ACAGACCGAT GACGTCGCCG GTGGACGGCT CAGTG

Sequence No.: 31

Length of sequence: 60

Type of sequence: Nucleic acid

Type of Chain : Single-stranded

Topology: Linear

Kind of sequence: Other nucleic acid; synthetic DNA

Features of sequence: P: Contains palindrome (GACGTC)

TGCGACCCCA GTGATTGACA GACCGATGAC GTCGCCGGTG GACGGCTCAG TGATAATTTA


Sequence No.: 32

Length of sequence: 80

Type of sequence: Nucleic acid

Type of Chain : Single-stranded

Topology: Linear

Kind of sequence: Other nucleic acid; synthetic DNA

Features of sequence: P: Contains palindrome (GACGTC)

CCTCAGTCAC TGCGACCCCA GTGATTGACA GACCGATGAC GTCGCCGGTG GACGGCTCAG

TGATAATTTA GATAGTACAC


Sequence No.: 33

Length of sequence: 45

Type of sequence: Nucleic acid

Type of Chain : Single-stranded

Topology: Linear

Kind of sequence: Other nucleic acid; synthetic DNA

Features of sequence: P: Contains palindrome (GACGTC)

ACCGATGACG TCGCGACGTC CGGCACCACG  ACGGCCACCG TGCTG


Sequence No.: 34

Length of sequence: 45

Type of sequence: Nucleic acid

Type of Chain : Single-stranded

Topology: Linear

Kind of sequence: Other nucleic acid; synthetic DNA

Features of sequence: P: Contains palindrome (GACGTC)

ACCGATGACG TCGCGACGTC CGGACGTCCG ACGGCCACCG TGCTG


Sequence No.: 35

Length of sequence: 45

Type of sequence: Nucleic acid

Type of Chain : Single-stranded

Topology: Linear

Kind of sequence: Other nucleic acid; synthetic DNA

Features of sequence: P: Contains palindrome (GACGTC)

ACCGATGACG TCGCGACGTC CGGACGTCCG GACGTCACCG TGCTG


Sequence No.: 36

Length of sequence: 45

Type of sequence: Nucleic acid

Type of Chain : Single-stranded

Topology: Linear

Kind of sequence: Other nucleic acid; synthetic DNA

Features of sequence: P: Contains palindrome (GACGTC)

ACCGATGACG TCGCGACGTC CGGACGTCCG GACGTCACCG ACGTC


Sequence No.: 37

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GACGTC)

GACGTCGACG TCGACGTCGA CGTCGACGTC


Sequence No.: 38

Length of sequence: 30

Type of sequence: Nucleic acid

Type of chain: Single-stranded

Topology: Linear

Kind of sequence: Other nucleic acid; synthetic DNA

Features of sequence: P: Contains palindrome (CACGTG)

CACGTGCACG TGCACGTGCA CGTGCACGTG


Sequence No.: 39

Length of sequence: 30

Type of sequence: Nucleic acid

Type of chain: Single-stranded

Topology: Linear

Kind of sequence: Other nucleic acid; synthetic DNA

Features of sequence: P: Contains palindrome (AACGTT)

AACGTTAACG TTAACGTTAA CGTTAACGTT

Sequence No.: 40

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GCGCGC)

GCGCGCGCGC GCGCGCGCGC GCGCGCGCGC

Sequence No.: 41

Length of sequence: 30

Type of sequence: Nucleic acid

Type of chain: Single-stranded

Topology: Linear

Kind of sequence: Other nucleic acid; synthetic DNA

Features of sequence: P: Contains palindrome (ATATAT)

ATATATATAT ATATATATAT ATATATATAT

Sequence No.: 42

Length of sequence: 45

Type of sequence: Nucleic acid

Type of chain: Double- stranded

Topology: Linear

Kind of sequence: Other nucleic acid; synthetic DNA

Features of sequence: P: Contains palindrome (GACGTC)

5'-ACCGATGACG TCGCCGGTGA CGGCACCACG ACGGCCACCG TGCTG-3'

3'-TGGCTACTGC AGCGGCCACT GCCGTGGTGC TGCCGGTGGC ACGAC-5'

Sequence No.:43

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GACGTC)

GGGGGGGGGG GGGACGTCGG GGGGGGGGGG

Sequence No.: 44

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GACGTC)

AAAAAAAAAA AAGACGTCAA AAAAAAAAAA

Sequence No.: 45

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GACGTC)

TTTTTTTTTT TTGACGTCTT TTTTTTTTTT


Sequence No.: 46

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GACGTC)

CCCCCCCCCC CCGACGTCCC CCCCCCCCCC


Sequence No.: 47

Length of sequence: 30

Type of sequence: Nucleic acid

Type of chain: Single-stranded

Topology: Linear

Kind of sequence: Other nucleic acid; synthetic DNA

Features of sequence: P: Contains palindrome(GACGTC)

GCGCGCGCGC GCGACGTCGC GCGCGCGCGC


Sequence No.: 48

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GACGTC)

GAGAGAGAGA GAGACGTCGA GAGAGAGAGA


Sequence No.: 49

Length of sequence: 30

Type of sequence: Nucleic acid

Type of chain: Single-stranded

Topology: Linear

Kind of sequence: Other nucleic acid; synthetic DNA

Features of sequence: P: Contains palindrome (CGATCG)

ACCGATCGAT CGGCCGGTGA CGGCACCACG


Sequence No.: 50

Length of sequence: 30

Type of sequence: Nucleic acid

Type of chain: Single-stranded

Topology: Linear

Kind of sequence: Other nucleic acid; synthetic DNA

Features of sequence: P: Contains palindrome (ATCGAT)

ACCGATATCG ATGCCGGTGA CGGCACCACG


Sequence No.: 51

Length of sequence: 30

Type of sequence: Nucleic acid

Type of chain: Single-stranded

Topology: Linear

Kind of sequence: Other nucleic acid; synthetic DNA

Features of sequence: P: Contains palindrome (TCGCGA)

ACCGATTCGC GAGCCGGTGA CGGCACCACG


Sequence No.: 52

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GCGCGC)

ACCGATGCGC GCGCCGGTGA CGGCACCACG


Sequence No.: 53

Length of sequence: 30

Type of sequence: Nucleic acid

Type of chain: Single-stranded

Topology: Linear

Kind of sequence: Other nucleic acid; synthetic DNA

Features of sequence: P: Contains palindrome (CGTACG)

ACCGATCGTA CGGCCGGTGA CGGCACCACG


Sequence No.: 54

Length of sequence: 30

Type of sequence: Nucleic acid

Type of chain: Single-stranded

Topology: Linear

Kind of sequence: Other nucleic acid; synthetic DNA

Features of sequence: P: Contains palindrome (AGCGCT)

ACCGATAGCG CTGCCGGTGA CGGCACCACG


Sequence No.: 55

Length of sequence: 30

Type of sequence: Nucleic acid

Type of chain: Single-stranded

Topology: Linear

Kind of sequence: Other nucleic acid; synthetic DNA

Features of sequence: P: Contains palindrome (CGGCCG)

ACCGATCGGC CGGCCGGTGA CGGCACCACG


Sequence No.: 56

Length of sequence: 30

Type of sequence: Nucleic acid

Type of chain: Single-stranded

Topology: Linear

Kind of sequence: Other nucleic acid; synthetic DNA

Features of sequence: P: Contains palindrome (GTCGAC)

ACCGATGTCG ACGCCGGTGA CGGCACCACG


Sequence No.: 57

Length of sequence: 30

Type of sequence: Nucleic acid

Type of chain: Single-stranded

Topology: Linear

Kind of sequence: Other nucleic acid; synthetic DNA

Features of sequence: P: Contains palindrome (CGCGCG)

ACCGATCGCG CGGCCGGTGA CGGCACCACG

Sequence No.: 58

Length of sequence: 30

Type of sequence: Nucleic acid

Type of chain: Single-stranded

Topology: Linear

Kind of sequence: Other nucleic acid; synthetic DNA

Features of sequence: P: Contains palindrome (ACGCGT)

ACCGATACGC GTGCCGGTGA CGGCACCACG

Sequence No.: 59

Length of sequence: 30

Type of sequence: Nucleic acid

Type of chain: Single-stranded

Topology: Linear

Kind of sequence: Other nucleic acid; synthetic DNA

Features of sequence: P: Contains palindrome (AAGCTT)

ACCGATAAGC TTGCCGGTGA CGGCACCACG

Sequence No.: 60

Length of sequence: 30

Type of sequence: Nucleic acid

Type of chain: Single-stranded

Topology: Linear

Kind of sequence: Other nucleic acid; synthetic DNA

Features of sequence: P: Contains palindrome (TTATAA)

ACCGATTTAT AAGCCGGTGA CGGCACCACG

Sequence No.: 61

Length of sequence: 30

Type of sequence: Nucleic acid

Type of chain: Single-stranded

Topology: Linear

Kind of sequence: Other nucleic acid; synthetic DNA

Features of sequence: P: Contains palindrome (TATATA)

ACCGATTATA TAGCCGGTGA CGGCACCACG

Sequence No.: 62

Length of sequence: 30

Type of sequence: Nucleic acid

Type of chain: Single-stranded

Topology: Linear

Kind of sequence: Other nucleic acid; synthetic DNA

Features of sequence: P: Contains palindrome (AGTACT)

ACCGATAGTA CTGCCGGTGA CGGCACCACG

Sequence No.: 63

Length of sequence: 30

Type of sequence: Nucleic acid

Type of chain: Single-stranded

Topology: Linear

42

Kind of sequence: Other nucleic acid; synthetic DNA

Features of sequence: P: Contains palindrome (GAATTC)

ACCGATGAAT TCGCCGGTGA CGGCACCACG


Sequence No.: 64

Length of sequence: 30

Type of sequence: Nucleic acid

Type of chain: Single-stranded

Topology: Linear

Kind of sequence: Other nucleic acid; synthetic DNA

Features of sequence: P: Contains palindrome (CTGCAG)

ACCGATCTGC AGGCCGGTGA CGGCACCACG


Sequence No.: 65

Length of sequence: 30

Type of sequence: Nucleic acid

Type of chain: Single-stranded

Topology: Linear

Kind of sequence: Other nucleic acid; synthetic DNA

Features of sequence: P: Contains palindrome (AAATTT)

ACCGATAAAT TGCCGGTGA CGGCACCACG


Sequence No.: 66

Length of sequence: 30

Type of sequence: Nucleic acid

Type of chain: Single-stranded

Topology: Linear

Kind of sequence: Other nucleic acid; synthetic DNA

Features of sequence: P: Contains palindrome (CCTAGG)

ACCGATCCTA GGGCCGGTGA CGGCACCACG

## Claims

1. An immunostimulatory remedy containing, as the active ingredient, a single-stranded, linear polydeoxyribonucleotide with a base number of 10 to 100 containing at least one structure represented by the following general formula:

   $$5'\text{-}X_n \bullet \bullet \bullet X_3 X_2 X_1 Y_1 Y_2 Y_3 \bullet \bullet \bullet Y_n\text{-}3' \qquad (I)$$

   ( wherein n is an integer from 3 to 50; $X_1$, $X_2$, $X_3$, $\bullet \bullet \bullet$, $X_n$ and $Y_1$, $Y_2$, $Y_3$, $\bullet \bullet \bullet$, $Y_n$ are each a monodeoxyribonucleotide; $X_1$, $X_2$, $X_3$, $\bullet \bullet \bullet$ and $X_n$ may be the same or different nucleotides; and bases in $X_1$ and $Y_1$, in $X_2$ and $Y_2$, in $X_3$ and $Y_3$, in $\bullet \bullet \bullet$, and in $X_n$ and $Y_n$ are complementary with each other as defined by Watson & Crick ) or a salt thereof.

2. An immunostimulatory remedy containing, as the active ingredient, a double-stranded, linear polydeoxyribonucleotide or a salt thereof, in which at least one single-stranded, linear polydeoxyribonucleotide contains at least one structure represented by the general formula (I).

3. An immunostimulatory remedy as described in Claim 1 or 2, wherein $X_1$ in the general formula (I) is deoxyguanylic acid or deoxycytidylic acid.

4. An immunostimulatory remedy as described in Claim 1 or 2, wherein the structure represented by the general formula (I) is 5'-GACGTC-3' ( wherein G is deoxyguanylic acid, A is deoxyadenylic acid, C is deoxycytidylic acid, and T is deoxythymidylic acid ).

5. An immunostimulatory remedy as described in Claim 1 or 2, wherein the structure represented by the general formula (I) is 5'-AGCGCT-3' ( wherein G is deoxyguanylic acid, A is deoxyadenylic acid, C is deoxycytidylic acid, and T is deoxythymidylic acid ).

6. An immunostimulatory remedy as described in Claim 1 or 2, wherein the structure represented by the general formula (I) is 5'-GATATC-3' ( wherein G is deoxyguanylic acid, A is deoxyadenylic acid, C is deoxycytidylic acid, and T is deoxythymidylic acid ).

7. An immunostimulatory remedy as described in Claim 1 or 2 , wherein the structure represented by the general formula (I) is 5'-AGGCCT-3' ( wherein G is deoxyguanylic acid, A is deoxyadenylic acid, C is deoxycytidylic acid, and T is deoxythymidylic acid ).

8. An immuostimulatory remedy as described in Claim 1 or 2, wherein the structure represented by the general formula (I) is 5'-ACGCGT-3' ( wherein G is deoxyguanylic acid, A is deoxyadenylic acid, C is deoxycytidylic acid, and T is deoxythymidylic acid ).

9. An immunostimulatory remedy as described in Claim 1 or 2, wherein the structure represented by the general formula (I) is 5'-CGATCG-3' ( wherein G is deoxyguanylic acid, A is deoxyadenylic acid, C is deoxycytidylic acid, and T is deoxythymidylic acid ).

10. An immunostimulatory remedy as described in Claim 1 or 2 , wherein the structure represented by the general formula (I) is 5'-ATCGAT-3' ( wherein G is deoxyguanylic acid, A is deoxyadenylic acid, C is deoxycytidylic acid, and T is deoxythymidylic acid ).

11. An immunostimulatory remedy as described in Claim 1 or 2 , wherein the structure represented by the general formula (I) is 5'-TCGCGA-3' ( wherein G is deoxyguanylic acid, A is deoxyadenylic acid, C is deoxycytidylic acid, and T is deoxythymidylic acid ).

12. An immunostimulatory remedy as described in Claim 1 or 2, wherein the structure represented by the general formula (I) is 5'-AACGTT-3' ( wherein G is deoxyguanylic acid, A is deoxyadenylic acid, C is deoxycytidylic acid, and T is deoxythymidylic acid ).

13. An immunostimulatory remedy as described in Claim 1 or 2 , wherein the structure represented by the general formula (I) is 5'-GCGCGC-3' ( wherein G is deoxyguanylic acid, and C is deoxycytidylic acid ).

14. An immunostimulatory remedy as described in Claim 1 or 2, wherein the structure represented by the general formula (I) is 5'-CGTACG-3' ( wherein G is deoxyguanylic acid, A is deoxyadenylic acid, C is deoxycytidylic acid, and T is deoxythymidylic acid ).

15. An immunostimulatory remedy as described in Claim 1 or 2 , wherein the structure represented by the general formula (I) is 5'-CGGCCG-3' ( wherein G is deoxyguanylic acid, and C is deoxycytidylic acid ).

16. An immunostimulatory remedy as described in Claim 1 or 2 , wherein the structure represented by the general formula (I) is 5'-GTCGAC-3' ( wherein G is deoxyguanylic acid, A is deoxyadenylic acid, C is deoxycytidylic acid, and T is deoxythymidylic acid ).

17. An immunostimulatory remedy as described in Claim 1 or 2 , wherein the structure represented by the general formula (I) is 5'-CGCGCG-3' ( wherein G is deoxyguanylic acid, and C is deoxycytidylic acid ).

18. An immunostimulatory remedy as described in any of Claims 1 through 17 , wherein the portion except the structure represented by the general formula (I) is the repeated structure of deoxyguanylic acid.

19. An immunostimulatory remedy as described in Claim 1 or 2 , wherein the structure represented by the general formula (I) contains at least one structure of 5'-CG-3' ( wherein G is deoxyguanylic acid, and C is deoxycytidylic acid ).

20. An immunostimulatory remedy as described in Claim 19 , wherein the portion except the structure represented by the generagl formula (I) is the repeated structure of deoxyguanylic acid.

21. An immunostimulatory remedy as described in any one of Claims 1 to 20, for the relief of malignant tumors, infectious dieases, immunodeficiency diseases and autoimmune diseases.

45